# EUROPEAN PATENT APPLICATION

(11) **EP 3 670 722 A1**
(43) Date of publication of application: **24.06.2020**
(21) Application number: 18846682.5
(22) Date of filing: 10.08.2018
(51) Int. Cl.: D03D 11/00, D03D 7/00, A61N 1/04, B32B 5/26

(54) **TEXTILE STRUCTURE FOR ATTACHING ELECTRODES TO ELASTIC FABRICS USED IN MUSCULAR ELECTROSTIMULATION GARMENTS**

(30) Priority: 14.08.2017 ES 201700599 U
(71) Applicant: Bylebron, S.L.U., 28037 Madrid (ES)
(72) Inventor: CARRILLO GONZALEZ, Beatriz, 28037 Madrid (ES)
(74) Representative: Sanz-Bermell Martinez, Alejandro
(86) International application number: PCT/ES2018/070553
(87) International publication number: WO 2019/034797

(57) **Abstract**

The invention relates to a textile structure comprising a fabric (5) formed of a first outer layer of a first textile material (1), a second inner layer of a second textile material (2), and a bonding layer (4) between the first layer and the second layer, a bonding layer of an adhesive medium or glue, wherein the first textile material (1) is an elastic material, and wherein the second textile material (2) is formed of one part (2') of two complementary parts (2', 2") of a self-adhesive textile material, the second textile material (2) also being elastic.

## Description

### Purpose of the invention

The purpose of this invention is a fabric structure, in particular an elastic fabric, which is light and breathable, used in making muscular electrostimulation garments, and the fastening of the electrodes used in these types of garments.

The purpose of the invention is to provide a fabric structure to which the user can fasten electrodes to electrostimulation garments at certain or predetermined positions, enabling these to be fastened firmly on the inside of the of the garment during use.

Also, an additional purpose of the invention is the use of fastening devices for the electrode power cables in the garment, which obviates the need to pierce the fabric or use other complicated means of fastening, all with a resistant, flexible, light and economical fabric.

### Background of the invention

Electrical muscle stimulation is a technology that induces muscle contractions with electrical impulses, which reach the motor nerve through the skin, reproducing the natural physiology of a contraction. This occurs due to an electrical circuit having closed over the body, which is the conductor or transmitter of the impulse to the body - the electrode. Electrostimulation has been used for decades for sports performance purposes, for therapeutic purposes and also for aesthetic purposes.

This system causes involuntary muscle contractions and depending on the context or objective should or should not be accompanied by voluntary activity, for instance, when we want to increase muscle mass or strength, or increase our caloric expenditure, this voluntary activity is necessary, but when it is applied for anti-algic purposes or recovery, it is not.

For the use of electrostimulation garments, normally the users wear undergarments which could be a bodysuit, a t-shirt and a culotte. The electrostimulation garments are placed over these undergarments.

The placement of electrodes for electrostimulation, nevertheless, can cause inconveniences for different reasons:
The first reason is that it is a slow and tedious process to place the electrodes in the first phase to later have to put on the garment in which they are fastened, and also having to connect each one or every group of electrodes;

Another disadvantage is that, in the process of putting on the electrostimulation garment and while using it, the electrodes can lose adherence to the user or patient or to the undergarments they are wearing or move out of place. And this means that the electrical impulse losses its effectiveness, as every muscle group has an optimum point of stimulation. In addition, when the contact surface is reduced, this can lead to significant discomfort.

Another drawback is that "loose" electrodes may not place themselves in suitable areas or perform their intended function, or their function may have a negative effect. ES 1 106 785 U describes an electrostimulation electrode for its dry application to the entire skin of a patient which may be used for this purpose.

To avoid the above-mentioned problems of affixing the electrodes directly onto the patient's skin, electrical stimulation garments have been developed that include electrodes already sewn into the inside face of the garment so that when the user wears that garment the electrodes are already in working position, noticeably reducing the preparation time.

Thus, a common practice in electrical stimulation techniques is that they are placed in garments that the user places for the corresponding session. ES 1 129 005 U refers to clothing used for electrical stimulation muscle training, which includes a set of electrodes in certain areas of the garment, and which provides a detachable textile cover. The garments tend to be elastic, so as to produce a reliable contact between the electrodes and the skin of the user. Elastic garments are normally made with a central neoprene layer with a textile cover on both sides, which has various disadvantages, such as great specific density and weight by unit area, little flexibility and no breathability.

Electrical stimulation garments include electrodes already sewn into the inside of the garment so that when the user dresses with that garment the electrodes are directly placed in a working position, noticeably reducing the preparation time. One problem with these garments is that because the electrodes are sewn in, their position cannot be changed, and this is not always ideal in terms of the anatomy of each user. To this the fact should be added that this stitched-in attachment weakens the garment material, as the sewing requires piercing it with a needle, and given that these garments are normally elastic materials, they are also more susceptible to tearing with any kind of hole in the fabric. The use of reinforcement to reduce the risk of tearing or deterioration means an increase in weight and stiffness.

Other materials used to make electrical stimulation garments are the so-called 3D materials, which, while they do provide a certain degree of breathability, have little or no elasticity and limited flexibility.

### Description of the invention

To resolve the above-mentioned problems, a textile structure is proposed to fasten electrodes in elastic fabrics for use in electrical stimulation garments, that include at least the following elements:
A fabric, made from:
- A first outer layer, of a first textile material:
- A second, inner layer, of a second textile material.
- A bonding layer between the first and second layers, unlike the fabrics used in electrical stimulation in which an intermediate layer of neoprene or similar material is placed in the fabrics, made from an adhesive substance or glue. The neoprene of the known previous fabrics forms a continuous layer which is not breathable, which is also a heavy and excessively rigid fabric, and is therefore uncomfortable to handle and use, store, wash, etc. The use of an adhesive or glue in the fabric of this invention binds together the fibers of the fabrics of the two layers, but does not fully cover the holes existing between the warp and weft of each layer, meaning, it does not create a continuous surface layer. For this reason, it has the advantage of being breathable, besides being flexible, adjustable and resistant; it also has good electrical insulation, so there will be no electrical derivations through the fabric. In addition, it is a fabric whose specific weight by surface unit is significantly lower than known fabrics.

And a group of electrodes provided in the surface opposite to the surface facing the user, of a third textile material.

In the second textile material of the second or inner layer and the third textile material fixed in the electrode constitute a system of textile bonding in which each of the said second and third textile materials is formed of one of two elements complementary to that textile bonding system, such as a system of hooks and loops, commonly known under the trade name Velcro ®.

The bonding layer between the first layer and the second layer consists of an adhesive.

The first layer or outer layer of the fabric is normally made of Lycra (also called "Spandex" or "Elastane") while the inner layer of fabric is an elastic material, whose inner surface is made of one of the parts of an adhesive textile material, for instance, of hooks and loops, in cooperation with the external surface of the third textile material on the electrodes. This enables fastening the electrostimulation electrodes at the points considered ideal at any time, without affecting the structure of the fabric, and allowing easy modification of its position, through mere separation of the layers and renewed placement.

The self-adhering materials are normally formed of two cooperating components, of a hook and loop fabric, where the first component has tiny hooks: the second incorporates even smaller and "furry" loop tape. By putting together the two components, the hooks are hooked onto loop tapes and the two parts are joined or fastened until they are pulled apart. Usually, the inner layer of the fabric of the electrical stimulation garment (the second textile material) will comprise on its inner surface the loops of the material, while the third textile material, positioned over each electrode, will comprise the hooks.

In this way, the fabric thus constituted is fully integrated in its entirety, both due to its elasticity, resistance, lightness and flexibility, ideal for electrostimulation clothing.

Preferably, the textile material that forms the first layer or outer layer of the fabric could be polyester-Lycra, or nylon-Lycra, in its different types of material, both in weft and warp and where the densities could range from 160 to 330 g/m2.

The second layer or inner layer may also have different densities and will preferably be between 54 and 320 g/m2.

The adherence of the first layer or outer layer and the second or inner layer (Lycra and elastic fabric with a self-adhesive material), results in a fabric that is very elastic and adjustable, comfortable and resistant, with a very sporty outer finish, while its interior, being self-adhesive, as mentioned above, provides a functional garment finish, which enables directly affixing the electrodes used in electrical stimulation without the need to have to sew them into the material, meaning the material is not harmed or pierced with a needle, giving a more resistant result for the garment.

The fabric is also perfectly washable and durable.

The adhesive used to bind the two layers will be a glue that is preferably applied at points, which allows for coating and making a breathable fabric, and it is an ecological and heat-resistant glue.

The layering of the glue on the fabric will be done at approximately 95°C in a very short time (less than one second), reducing the temperature by up to 40°C.

The amount of glue used will be approximately between 4 and 10 g/m2. Finally, the two layers that form the electrical stimulation garments will be high quality and have grade 4 heat resistance.

The scope of the invention also proposes the use of portions of the third textile material in the shape of strips or bands, which adhere to the inside of the second layer (facing the user) to support and hold in place the electrode connection cables, in addition to supporting those electrodes.

An advantageous feature which this invention resolves is that the fabric used to manufacture the garment is a good electrical insulator, such that the electricity of the electrodes cannot be transmitted through the garment's fabric, which electrically insulates them.

Another advantageous feature of the fabric is that it has antibacterial properties, which ensures better hygienic conditions of the garment in comparison with others without this feature, enabling a longer period of use of a single user between washes.

These characteristics of the fabric enable obtaining a fabric that in addition is flexible, highly resistant, breathable, comfortable, and soft and pleasant to the touch.

### Description of the drawings

To illustrate the following explanation, two-pages of descriptive drawings are attached, with three figures that represent, by way of a non-exhaustive example, the essence of the invention, and in which the following can be observed:

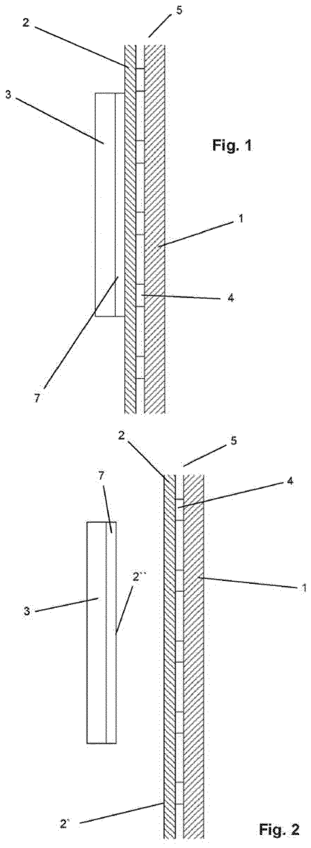

- Figure 1: Shows a schematic cross-section view of the invention's textile structure, with an electrode placed;
- Figure 2: Shows a schematic cross-section view, similar to figure 1, which shows the electrode in position separated from the fabric that holds it; and

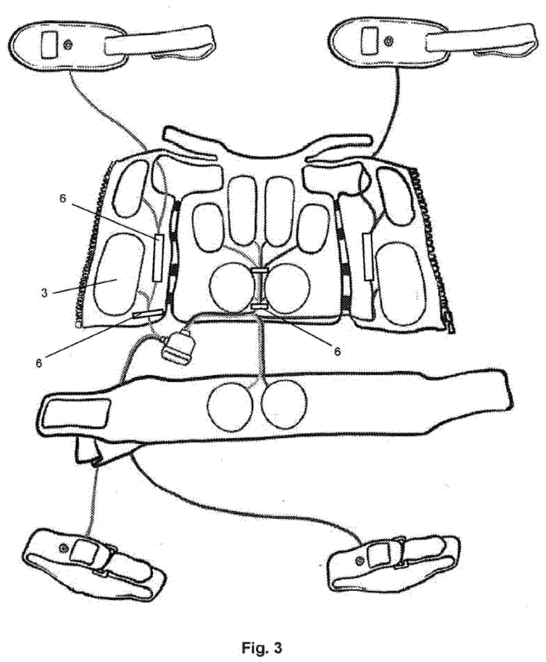

- Figure 3: Shows a schematic view of a garment carrying electrodes that uses the textile structure of figures 1 and 2.

These refer to the following reference signs:
1 first textile material, outer layer
2 second textile material, inner layer, provided with one of the two complementary parts of a self-adhesive textile material.
2' one of the two complementary parts of a self-adhesive textile material
2" the other of the two complementary parts of the self-adhesive textile electrodes
4 adhesive, glue or bonding layer between the first textile material and the second textile material
5 fabric formed by the first textile material and the second textile material, joined by the adhesive or bonding layer
6 strips or bands supporting the electrode cables
7 third textile material, with one of the two complementary parts of a self-adhesive textile material

### Preferred embodiment of the invention

These figures show the textile structure for fastening electrodes in elastic fabrics for use in electrostimulation garments, they show a fabric that (5) comprises two layers joined to each other with an adhesive. In particular the fabric (5) is formed of a first textile material (1) preferably elastic, which forms an outer layer, of Lycra or an equivalent material or with similar characteristics and a second textile material (2) which forms an inner layer of elastic fabric with a self-adhesive material. It is anticipated that the first textile material will be made of one or more of the materials: Lycra, nylon and/ or polyester. And in particular combinations Lycra-polyester or Lycra-nylon, in which the Lycra is woven in warp and weft. The first textile material (1) and the second textile material (2) are joined together with a bonding layer of an adhesive or glue (4). The adhesive layer is preferably discontinuous, for instance through small points, which maintains the breathability of the fabric (5). The second textile material (2) is composed of an elastic fabric whose inner surface (user-facing) is made, in turn, of one (2') of the two complementary parts of the self-adhesive textile material (normally a loop fabric). On the other hand, depending on the structure of the invention, the part behind the electrodes (3), that is to say, on the surface opposite the surface facing the user, is a third textile material (7), with the other (2') complementary part of the textile materials (normally micro-hooks) facing the outside. This configuration enables directly fastening the electrodes (3) to the inner layer by means of a textile adhesive between the two complementary parts (2', 2") of the self-adhesive textile material. In this way the electrodes (3) can be distributed over the fabric (the garment) in the way deemed suitable, without the need to sew and consequently damage, the fabric as traditionally occurs.

The Lycra that makes up the outer layer can be polyester-Lycra or nylon-Lycra, in its different possible types of fabric, with a density that can vary between 160 and 330 g/m2, while the inner layer (2) forms a layer the density of which can be between 54 and 320 g/m2, applying the glue (4) using small fixing points in alignment, in order to define breathing spaces for the fabric, which will be very elastic, comfortable and resistant, with a very sporty exterior finish.

The invention also includes strips or bands (6) of the third textile material (7), which can be directly applied to the inside of the garment to attach the electrode connection cables (3), avoiding the obvious discomfort these cables cause when loose. These strips can be narrow and hold the cables crossways at different points along the cable route, or wider and hold the cables along the entire route.

## Claims

1. Textile structure for fastening electrodes on elastic fabrics for use in electrical stimulation garments **characterised by** being a fabric (5) that is made with:
• A first outer layer of a first textile material (1);
• A second inner layer of a second textile material (2);
• A bonding layer (4) between the first layer and the second layer, of an adhesive or glue
in which the first textile material (1) is an elastic material; and
in which the second textile material (2) is formed of one (2') of the two complementary parts (2', 2") of the self-adhesive textile material, this second textile material (2) also being elastic.

2. Textile structure for fastening electrodes in elastic fabrics for use in electrical stimulation garments, according to claim 1, **characterised by** electrodes (3), on the surface opposite the surface facing the user, is provided with the other (2") of the two complementary parts (2',2') of the self-adhesive textile material.

3. Textile structure for fastening electrodes in elastic fabrics for use in electrical stimulation garments, according to both claims 1 and 2, **characterised by** the material of the outer layer (1) being at least one of the following:
• Polyester
• Lycra
• Nylon
• Any combination of the above.

4. Textile structure for fastening electrodes in elastic fabrics for use in electrical stimulation garments, according to claim 3, **characterised by** the Lycra of the first textile material (1) is weft knitted.

5. Textile structure for fastening electrodes in elastic fabrics for use in electrical stimulation garments, according to claim 3 characterised because the Lycra of the first textile material (1) is warp knitted.

6. Textile structure for fastening electrodes in elastic fabrics for use as electrical stimulation garments, according to any of the claims from 1 to 5, characterised because the first textile material, the outer layer (1), has a weight between 160 and 330 g/m2.

7. Textile structure for fastening electrodes in elastic fabrics for use in electrical stimulation garments, according to any of the claims from 1 to 6, characterised because the second textile material (2), the inner layer, has a density between 54 and 320 g/m2.

8. Textile structure for fastening electrodes in elastic fabrics for use as electrical stimulation garments, according to any of the claims from 1 to 7 characterised because the binding layer (4) that joins the first textile material (1) and the second textile material (2) is discontinuous.

9. Textile structure for fastening electrodes in elastic fabrics for use as electrical stimulation garments, according to claim 8, characterised because the binding layer (3) that joins the first textile material (1) and the second textile material (2) is formed of small points between which there are spaces for the fabric to breathe.

10. Textile structure for fastening electrodes in elastic fabrics for use in electrical stimulation garments, according to claim 8, characterised because it also includes strips or bands (6) of a third textile material (7), to secure the electrode connection cables (3).

11. Textile structure for fastening electrodes in elastic fabrics for use as electrical stimulation garments, according to any of the claims from 3 to 10, **characterised by** the garment fabric being electrically insulated.

12. Textile structure for fastening electrodes in elastic fabrics for use as electrical stimulation garments, according to any of the claims from 3 to 11, **characterised by** the garment fabric being anti-bacterial.
